# EUROPÄISCHE PATENTSCHRIFT

(11) **EP 3 512 847 B1**
(45) Veröffentlichungstag und Bekanntmachung des Hinweises auf die Patenterteilung: **30.03.2022**
(21) Anmeldenummer: 17765149.4
(22) Anmeldetag: 06.09.2017
(51) Int. Cl.: C07D 471/04, C07D 487/04, C07F 3/06

(54) **VERFAHREN ZUR HERSTELLUNG VON HALOGENIERTEN BIZYKLEN**
PROCESS FOR THE PREPARATION OF HALOGENATED BICYCLES
PROCÉDÉ POUR LA PREPARATION DES BICYCLES HALOGÉNÉS

(30) Priorität: 14.09.2016 EP 16188760
(43) Veröffentlichungstag der Anmeldung: 24.07.2019
(73) Patentinhaber: Bayer CropScience Aktiengesellschaft, 40789 Monheim am Rhein (DE)
(72) Erfinder: MOSRIN, Marc, 50935 Köln (DE); FISCHER, Rüdiger, 50259 Pulheim (DE); HAGER, Dominik, 40789 Monheim (DE); HOFFMEISTER, Laura, 40593 Düsseldorf (DE); KAUSCH-BUSIES, Nina, 51467 Bergisch Gladbach (DE); WILCKE, David, 40235 Düsseldorf (DE); WILLOT, Matthieu, 40215 Düsseldorf (DE)
(74) Vertreter: BIP Patents
(86) Internationale Anmeldenummer: PCT/EP2017/072363
(87) Internationale Veröffentlichungsnummer: WO 2018/050515

(56) Entgegenhaltungen:
- EP-A1- 2 857 396
- ROBERT J. WILSON ET AL: "Copper- and Palladium-Catalyzed Amidation Reactions for the Synthesis of Substituted Imidazo[4,5- c ]pyridines", THE JOURNAL OF ORGANIC CHEMISTRY, Bd. 79, Nr. 5, 7. März 2014 (2014-03-07), Seiten 2203-2212, XP055113503, ISSN: 0022-3263, DOI: 10.1021/jo500064j in der Anmeldung erwähnt
- SAJITH AYYILIATH M ET AL: "Microwave enhanced Suzuki coupling: a diversity-oriented approach to the synthesis of highly functionalised 3-substituted-2-aryl/heteroaryl imidazo[4,5-b]pyridines", TETRAHEDRON LETTERS, Bd. 53, Nr. 9, 2012, Seiten 1036-1041, XP028887817, ISSN: 0040-4039, DOI: 10.1016/J.TETLET.2011.12.051 in der Anmeldung erwähnt

## Beschreibung

Die vorliegende Erfindung betrifft ein Verfahren zur Herstellung von halogenierten Bizyklen der Formel (II)

Q-Hal (II),ausgehend von Verbindungen Q-H über Zwischenstufen der Formel (IIIa) oder (IIIb) 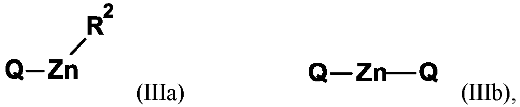 in denen die in den Formeln Q-H, (II), (IIIa) und (IIIb) angegebenen Strukturelemente die nachstehend angegebenen Bedeutungen haben. Ferner betrifft die Erfindung derartige halogenierte Bizyklen und Zwischenstufen.

Halogenierte Bizyklen der Formel (II) haben große technische Bedeutung für die pharmazeutische und die agrochemische Industrie und sind eine wichtige Zwischenstufe unter anderem bei der Herstellung von Verbindungen, die beispielsweise als Pestizide wirksam sind.

In der Literatur ist bekannt, dass Verbindungen der Formel (II) in einem ersten Schritt durch Metallierung in Gegenwart einer sehr reaktiven Lithium-Base wie beispielsweise Lithiumdiisopropylamid oder *n*-Butyllithium und anschließender Umsetzung mit einer Halogenidquelle wie beispielsweise Hexachlorethan oder N-Iodsuccinimid (wie in Journal of Organic Chemistry 2014, 79, 2203-2212 und Tetrahedron Letters 2012, 53, 1036-1041 beschrieben) hergestellt werden können. Ebenfalls ist bekannt, dass Verbindungen der Formel (II) durch Reaktion von Hydroxybizyklen mit Phosphortrichlorid (wie in WO 2013/180193 beschrieben) hergestellt werden können. Die bisher im Stand der Technik beschriebenen chemischen Syntheseverfahren von derartigen halogenierten Bizyklen bedienen sich aber sehr häufig Methoden, die aus industrieller Sicht wirtschaftlich nicht zu realisieren sind und/oder andere Nachteile aufweisen.

Nachteilig sind die geringen chemischen Ausbeuten vor allem bei hoch substituierten Bizyklen, die Durchführung bei sehr tiefen Temperaturen für Metallierungen (ca. -80°C) sowie die in der Regel schwierige Regio- und Chemo-Selektivität der Halogenierung. Darüber hinaus ist die Einführung von Brom- oder insbesondere von Iod-Atomen in derartige Hydroxybizyklen generell problematisch oder bislang überhaupt nicht möglich. Die Herstellung ist - wenn überhaupt möglich - daher sehr teuer und nicht für großtechnische kommerzielle Verfahren geeignet. Außerdem sind entsprechende Verbindungen kaum kommerziell erhältlich. Dies gilt insbesondere für 7-Methyl-7H-imidazo[4,5-c]pyridazin, 3-Methyl-3H-imidazo[4,5-c]pyridin und 3-Methyl-3H-imidazo[4,5-b]pyridin.

Im Hinblick auf die vorstehend geschilderten Nachteile besteht dringend Bedarf für ein vereinfachtes, technisch und ökonomisch durchführbares Verfahren zur Herstellung von halogenierten Bizyklen, insbesondere von halogenierten Bizyklen der Formel (II). Die mit diesem angestrebten Verfahren erhältlichen halogenierten Bizyklen sollen dabei vorzugsweise mit guter Ausbeute, hoher Reinheit und in ökonomischer Weise erhalten werden.

Überraschenderweise wurde gefunden, dass in einem Verfahren unter Verwendung einer Zink-metallorganischen Base halogenierte Bizyklen der Formel (II) vorteilhaft hergestellt werden können.

Gegenstand der vorliegenden Erfindung ist demnach ein Verfahren zur Herstellung von Verbindungen der Formel (II)

Q-Hal (II),

in welcher (Ausgestaltung 1)
- Q: für ein Strukturelement 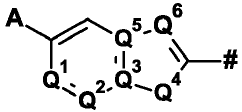
steht, wobei das Zeichen # die Bindung zu Hal anzeigt und
- Q¹: für N oder CR⁶ steht,
- Q²: für N oder CR⁶ steht,
- Q³: für N oder C steht,
- Q⁴: für O, S, N oder NR⁷ steht,
- Q⁵: für N oder C steht,
- Q⁶: für N oder CH steht,
und wobei Q für ein Strukturelement aus der Reihe Q2, Q3, Q10, Q12 oder Q14 steht, 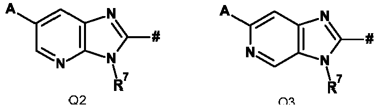 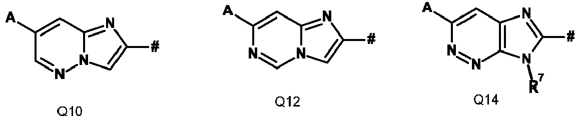
- R⁶: für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
- R⁷: für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
- A: für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthₗo, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkylaminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl steht,

Hal für Halogen steht,
dadurch gekennzeichnet, dass in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher
R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 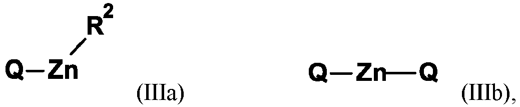
in welcher Q und R² jeweils die oben genannten Bedeutungen haben,
und diese Verbindung der Formel (IIIa) oder (IIIb) in einem zweiten Verfahrensschritt b) mit einer Verbindung der Struktur X-Hal, in welcher X für Halogen steht und Hal die oben genannte Bedeutung hat, zur Verbindung der Formel (II) umgesetzt wird.

Bei der Verbindung X-Hal handelt es sich, wie aus den Definitionen für X und Hal hervorgeht, um eine Interhalogenverbindung, vorzugsweise um elementares Halogen.

Vorzugsweise stehen Q¹, Q², Q³, Q⁴, Q⁵ und Q⁶ für nicht mehr als insgesamt fünf Stickstoffatome und weiter bevorzugt für nicht mehr als insgesamt vier Stickstoffatome.

Bevorzugte und besonders bevorzugte Bedeutungen der in den vorstehend erwähnten Formeln Q-H, (II), (IIIa) und (IIIb) des erfindungsgemäßen Verfahrens aufgeführten Reste Q, X, Hal und R² werden im Folgenden erläutert, wobei die Zink-metallorganische Base weiter unten noch genau beschrieben wird, so dass die bevorzugten Ausgestaltungen der Base dort angegeben sind.

### (Ausgestaltung 2)

- Q: steht bevorzugt für ein Strukturelement aus der Reihe Q2, Q3, Q10, Q12 oder Q14, 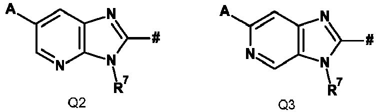 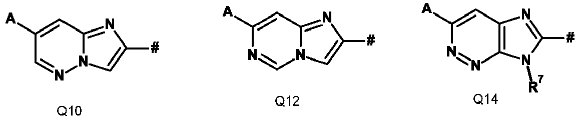
- R⁷: steht bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
- A: steht bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl,
- Hal: und X haben dieselbe Bedeutung und stehen bevorzugt für Fluor, Chlor, Iod oder Brom, und
- R²: steht bevorzugt für Halogen, insbesondere Chlor, Brom oder Iod,

### (Ausgestaltung 3)

- Q: steht besonders bevorzugt für ein Strukturelement aus der Reihe Q2, Q3, Q10, Q12 oder Q14,
- R⁷: steht besonders bevorzugt für (C₁-C₄)Alkyl oder (C₁-C₄)Alkoxy-(C₁-C₄)alkyl,
- A: steht besonders bevorzugt für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl,
- Hal: und X haben dieselbe Bedeutung und stehen besonders bevorzugt für Iod oder Brom, und
- R²: steht besonders bevorzugt für Chlor,

### (Ausgestaltung 4)

- Q: steht ganz besonders bevorzugt für das Strukturelement Q2, Q3, Q12 oder Q14,
- R⁷: steht ganz besonders bevorzugt für Methyl, Ethyl, n-Propyl oder iso-Propyl, insbesondere Methyl,
- A: steht ganz besonders bevorzugt für Trifluormethyl,
- Hal: und X haben dieselbe Bedeutung und stehen ganz besonders bevorzugt für Iod oder Brom, und
- R²: steht ganz besonders bevorzugt für Chlor.

Die vorstehend aufgeführten Restedefinitionen bzw. Erläuterungen gelten sowohl für die Endprodukte und Zwischenprodukte als auch für die Ausgangsprodukte entsprechend. Diese Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

In einer weiteren bevorzugten Ausführungsform der Erfindung steht Q für Q2 und R⁷, A, Hal, X und R² haben die in Ausgestaltung 1 oder die in Ausgestaltung 2 oder die in Ausgestaltung 3 oder die in Ausgestaltung 4 angegebenen Bedeutungen (Ausgestaltung 6).

In einer weiteren bevorzugten Ausführungsform der Erfindung steht Q für Q3 und R⁷, A, Hal, X und R² haben die in Ausgestaltung 1 oder die in Ausgestaltung 2 oder die in Ausgestaltung 3 oder die in Ausgestaltung 4 angegebenen Bedeutungen (Ausgestaltung 7).

In einer weiteren bevorzugten Ausführungsform der Erfindung steht Q für Q10 und R⁷, A, Hal, X und R² haben die in Ausgestaltung 1 oder die in Ausgestaltung 2 oder die in Ausgestaltung 3 oder die in Ausgestaltung 4 angegebenen Bedeutungen (Ausgestaltung 14).

In einer weiteren bevorzugten Ausführungsform der Erfindung steht Q für Q12 und R⁷, A, Hal, X und R² haben die in Ausgestaltung 1 oder die in Ausgestaltung 2 oder die in Ausgestaltung 3 oder die in Ausgestaltung 4 angegebenen Bedeutungen (Ausgestaltung 16).

In einer weiteren bevorzugten Ausführungsform der Erfindung steht Q für Q14 und R⁷, A, Hal, X und R² haben die in Ausgestaltung 1 oder die in Ausgestaltung 2 oder die in Ausgestaltung 3 oder die in Ausgestaltung 4 angegebenen Bedeutungen (Ausgestaltung 18).

Vorteilhafterweise lassen sich die halogenierten Bizyklen der Formel (II) mit dem erfindungsgemäßen Verfahren mit guten Ausbeuten und in hoher Reinheit herstellen. Ein großer Vorteil des erfindungsgemäßen Verfahrens ist dessen Regioselektivität und die vergleichsweise milden Reaktionsbedingungen, unter denen es durchgeführt werden kann, wesentlich bedingt durch die Durchführbarkeit bei deutlich höheren Temperaturen im Vergleich mit -80°C. Die Möglichkeit, auch bei deutlich höheren Temperaturen Halogene einführen zu können, ist sehr attraktiv, wobei in erfindungsgemäßen Verfahren auch bei derartigen höheren Temperaturen funktionelle Gruppen wie Trifluormethyl- oder andere elektronenziehende Gruppen, welche *ortho*-Positionen aktivieren, toleriert werden, ohne die vorhandene Regioselektivität zu beeinträchtigen. Ferner sind aufgrund der sehr guten funktionellen Gruppen-Toleranz von Zink-Reagenzien Zinkbasen sehr attraktiv. Insgesamt können somit Verbindungen der Formel (II) in kurzer Zeit und sehr guten Ausbeuten hergestellt werden.

Das erfindungsgemäße Verfahren kann anhand des folgenden Schemas (I) erläutert werden:

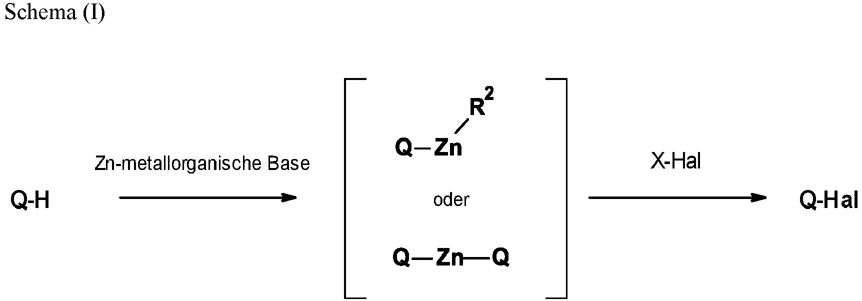

Hierin haben Q, X, Hal und R² sowie die innerhalb der jeweiligen Definitionen ggfs. vorhandenen weiteren Strukturelemente jeweils die vorstehend angegebenen Bedeutungen. Die in Klammern angegebenen Verbindungen stellen die Zwischenstufe (Formel IIIa beziehungsweise IIIb) dar, welche mit einer Verbindung X-Hal weiter zur Verbindung der Formel (II) umgesetzt wird. Demnach lässt sich das erfindungsgemäße Verfahren in die beiden Verfahrensschritte a) und b) unterteilen, wobei Schritt a) die Umsetzung der Verbindung Q-H zur jeweiligen Zwischenstufe und Schritt b) die weitere Umsetzung der Zwischenstufe zur Verbindung der Formel (II) ist.

### Allgemeine Definitionen

Im Zusammenhang mit der vorliegenden Erfindung umfasst der Begriff Halogen (Hal), soweit nicht anders definiert, solche Elemente, die ausgewählt sind aus der Gruppe bestehend aus Fluor, Chlor, Brom und Iod.

Der Begriff "Halogenide" beschreibt im Zusammenhang mit der vorliegenden Erfindung Verbindungen zwischen Halogenen und Elementen anderer Gruppen des Periodensystems, wobei salzartige Halogenide (ionische Verbindungen (Salze), die aufgrund der großen Elektronegativitätsdifferenz zwischen den beteiligten Elementen aus Anionen und Kationen bestehen und durch elektrostatische Wechselwirkungen zusammengehalten werden) oder kovalente Halogenide (kovalente Verbindungen, bei denen der Elektronegativitätsunterschied nicht so groß ist wie bei den vorstehend genannten ionischen Verbindungen, die Bindungen jedoch eine Ladungspolarität aufweisen) vorliegen können, abhängig von der Art der chemischen Bindung. Erfindungsgemäß besonders bevorzugt sind salzartige Halogenide.

Der Begriff "Pivaloyl" beschreibt im Zusammenhang mit der vorliegenden Erfindung den deprotonierten Rest der Pivalinsäure (X) mit der Summenformel (CH₃)₃CCO₂H. 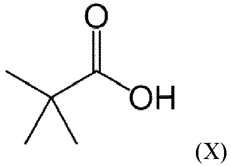 "O-Pivaloyl" bedeutet entsprechend, dass die Bindung des Pivaloylrestes über das deprotonierte Sauerstoffatom der Säuregruppe erfolgt.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste .

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Soweit nicht anders definiert steht Halogen dabei für Fluor, Chlor, Brom oder Iod, insbesondere für Fluor, Chlor oder Brom. Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂ oder CF₃CCl₂.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die Synthese von Verbindungen Q-H als Edukte eines erfindungsgemäßen Verfahrens ist dem Fachmann grundsätzlich bekannt. Beispielsweise können Verbindungen Q-H mit Q = Q1, Q2, Q3, Q14 oder Q15 aus entsprechenden Pyridin-Diaminderivaten durch Ringschluss zur jeweiligen Azolverbindung erhalten werden, wie zum Beispiel in WO2014/100065 oder WO2015/017610 beschrieben, vorzugsweise unter sauren Bedingungen. Alternativsynthesen sind ebenfalls möglich, sind jedoch komplexer und dadurch im Regelfall wirtschaftlich nachteiliger.

Die Umsetzung der Verbindungen Q-H zu Verbindungen der Formel (IIIa) oder Formel (IIIb) im ersten Verfahrensschritt (Schritt a)) erfolgt in Gegenwart einer Zink-metallorganischen Base der Struktur (NR³R⁴)-Zn-R² oder (NR³R⁴)₂-Zn, in welcher (Ausgestaltung B-1)
R² wie vorstehend (Ausgestaltung 1) definiert ist (daher für Halogen oder -O-Pivaloyl steht),
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl, Ethyl, n-Propyl und i-Propyl.

Bevorzugt ist, dass (Ausgestaltung B-2)
R² wie vorstehend als bevorzugt (Ausgestaltung 2) definiert ist (daher für Halogen steht, insbesondere für Chlor, Brom oder Iod),
R³ und R⁴ gemeinsam eine -(CH₂)₅-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl und Ethyl.

Besonders bevorzugt ist, dass (Ausgestaltung B-3)
R² wie vorstehend als besonders bevorzugt (Ausgestaltung 3) oder als ganz besonders bevorzugt (Ausgestaltung 4) definiert ist (daher für Chlor steht) und
R³ und R⁴ gemeinsam eine -(CH₂)₅-Gruppe bilden, die durch 4 Methylgruppen substituiert ist.

Die vorstehend aufgeführten Restedefinitionen können untereinander, also auch zwischen den jeweiligen Vorzugsbereichen, beliebig kombiniert werden.

In einer ganz besonders bevorzugten Ausgestaltung der erfindungsgemäßen Base ist das Strukturelement (NR³R⁴) Tetramethylpiperidin (TMP) gemäß Formel (IV).

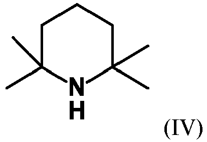

Erfindungsgemäß ganz besonders bevorzugte Zink-metallorganische Basen sind demnach dadurch gekennzeichnet, dass Zink gebunden an TMP vorliegt, insbesondere als Zinkhalogenid und ganz besonders bevorzugt als Zinkchlorid. Derartige Basen weisen folgende Struktur der Formel (V) auf (Ausgestaltung B-4)

(V) (TMP)ₓZnCl₂₋ₓ,

worin x für die Zahl 1 oder 2 steht. Hierunter wiederum bevorzugt sind Basen mit x=1 (Ausgestaltung B-5) gemäß Formel (VI):

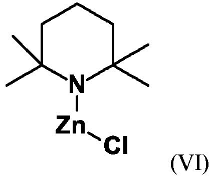

In einer weiteren bevorzugten Ausführungsform des erfindungsgemäßen Verfahrens liegt die Zink-metallorganische Base in Verbindung mit Alkali- oder Erdalkalihalogeniden vor. Dies gilt insbesondere für Basen der Formeln (V) und (VI). Besonders bevorzugte derartige Alkali- oder Erdalkalihalogenide sind Lithiumchlorid und Magnesiumchlorid, ganz besonders bevorzugt ist Lithiumchlorid. Erfindungsgemäß ganz besonders bevorzugte Zink-metallorganische Basen sind demnach TMP ZnCl·LiCl oder (TMP)₂ Zn·2LiCl (Ausgestaltung B-6). Am meisten bevorzugt ist TMP ZnCl·LiCl (VII; Ausgestaltung B-7).

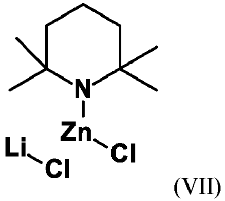

Beispielhaft werden in nachfolgender Tabelle 1 konkrete Kombinationen von Verbindungen der Formeln Q-H, (II) und (IIIa) bzw. (IIIb) mit erfindungsgemäßen Basen genannt, die in einem erfindungsgemäßen Verfahren zur Anwendung kommen können. Da in einigen Ausgestaltungen das Strukturelement R² sowohl in der erfindungsgemäßen Base als auch in der Verbindung der Formel (IIIa) vorliegt, gilt für R² jeweils die engste Definition.

**Tabelle 1:**

| Nummer | Verbindungen der Formeln Q-H, (II) und (IIIa) bzw. (IIIb) | Base gemäß |
|---|---|---|
| 1 | Ausgestaltung 1 | Ausgestaltung B-1 |
| 2 | Ausgestaltung 1 | Ausgestaltung B-2 |
| 3 | Ausgestaltung 1 | Ausgestaltung B-3 |
| 4 | Ausgestaltung 1 | Ausgestaltung B-4 |
| 5 | Ausgestaltung 1 | Ausgestaltung B-5 |
| 6 | Ausgestaltung 1 | Ausgestaltung B-6 |
| 7 | Ausgestaltung 1 | Ausgestaltung B-7 |
| 8 | Ausgestaltung 2 | Ausgestaltung B-1 |
| 9 | Ausgestaltung 2 | Ausgestaltung B-2 |
| 10 | Ausgestaltung 2 | Ausgestaltung B-3 |
| 11 | Ausgestaltung 2 | Ausgestaltung B-4 |
| 12 | Ausgestaltung 2 | Ausgestaltung B-5 |
| 13 | Ausgestaltung 2 | Ausgestaltung B-6 |
| 14 | Ausgestaltung 2 | Ausgestaltung B-7 |
| 15 | Ausgestaltung 3 | Ausgestaltung B-1 |
| 16 | Ausgestaltung 3 | Ausgestaltung B-2 |
| 17 | Ausgestaltung 3 | Ausgestaltung B-3 |
| 18 | Ausgestaltung 3 | Ausgestaltung B-4 |
| 19 | Ausgestaltung 3 | Ausgestaltung B-5 |
| 20 | Ausgestaltung 3 | Ausgestaltung B-6 |
| 21 | Ausgestaltung 3 | Ausgestaltung B-7 |
| 22 | Ausgestaltung 4 | Ausgestaltung B-1 |
| 23 | Ausgestaltung 4 | Ausgestaltung B-2 |
| 24 | Ausgestaltung 4 | Ausgestaltung B-3 |
| 25 | Ausgestaltung 4 | Ausgestaltung B-4 |
| 26 | Ausgestaltung 4 | Ausgestaltung B-5 |
| 27 | Ausgestaltung 4 | Ausgestaltung B-6 |
| 28 | Ausgestaltung 4 | Ausgestaltung B-7 |
| 36 | Ausgestaltung 6 | Ausgestaltung B-1 |
| 37 | Ausgestaltung 6 | Ausgestaltung B-2 |
| 38 | Ausgestaltung 6 | Ausgestaltung B-3 |
| 39 | Ausgestaltung 6 | Ausgestaltung B-4 |
| 40 | Ausgestaltung 6 | Ausgestaltung B-5 |
| 41 | Ausgestaltung 6 | Ausgestaltung B-6 |
| 42 | Ausgestaltung 6 | Ausgestaltung B-7 |
| 43 | Ausgestaltung 7 | Ausgestaltung B-1 |
| 44 | Ausgestaltung 7 | Ausgestaltung B-2 |
| 45 | Ausgestaltung 7 | Ausgestaltung B-3 |
| 46 | Ausgestaltung 7 | Ausgestaltung B-4 |
| 47 | Ausgestaltung 7 | Ausgestaltung B-5 |
| 48 | Ausgestaltung 7 | Ausgestaltung B-6 |
| 49 | Ausgestaltung 7 | Ausgestaltung B-7 |
| 92 | Ausgestaltung 14 | Ausgestaltung B-1 |
| 93 | Ausgestaltung 14 | Ausgestaltung B-2 |
| 94 | Ausgestaltung 14 | Ausgestaltung B-3 |
| 95 | Ausgestaltung 14 | Ausgestaltung B-4 |
| 96 | Ausgestaltung 14 | Ausgestaltung B-5 |
| 97 | Ausgestaltung 14 | Ausgestaltung B-6 |
| 98 | Ausgestaltung 14 | Ausgestaltung B-7 |
| 106 | Ausgestaltung 16 | Ausgestaltung B-1 |
| 107 | Ausgestaltung 16 | Ausgestaltung B-2 |
| 108 | Ausgestaltung 16 | Ausgestaltung B-3 |
| 109 | Ausgestaltung 16 | Ausgestaltung B-4 |
| 110 | Ausgestaltung 16 | Ausgestaltung B-5 |
| 111 | Ausgestaltung 16 | Ausgestaltung B-6 |
| 112 | Ausgestaltung 16 | Ausgestaltung B-7 |
| 120 | Ausgestaltung 18 | Ausgestaltung B-1 |
| 121 | Ausgestaltung 18 | Ausgestaltung B-2 |
| 122 | Ausgestaltung 18 | Ausgestaltung B-3 |
| 123 | Ausgestaltung 18 | Ausgestaltung B-4 |
| 124 | Ausgestaltung 18 | Ausgestaltung B-5 |
| 125 | Ausgestaltung 18 | Ausgestaltung B-6 |
| 126 | Ausgestaltung 18 | Ausgestaltung B-7 |

Vorzugsweise wird die Zink-metallorganische Base in dem erfindungsgemäßen Verfahren in einer Gesamtmenge von 0,5 bis 5 Äquivalenten, vorzugsweise von 0,8 bis 2 Äquivalenten, weiter bevorzugt von 1 bis 1,5 Äquivalenten und besonders bevorzugt von 1,0 bis 1,2 Äquivalenten, bezogen auf die Verbindung Q-H, eingesetzt. Ein Vorteil des erfindungsgemäßen Verfahrens ist es diesbezüglich, dass die metallorganische Base in nahezu stöchiometrischen Mengen eingesetzt werden kann.

In Abhängigkeit davon, ob in der eingesetzten Zink-metallorganischen Base das Strukturelement (NR³R⁴) einfach oder zweifach vorhanden ist, entstehen Zwischenverbindungen der Formel (IIIa) beziehungsweise der Formel (IIIb) in Verfahrensschritt a).

Die Umsetzung der Verbindungen der Formel (IIIa) beziehungsweise (IIIb) zu Verbindungen der Formel (II) im zweiten Verfahrensschritt (Schritt b)) erfolgt in Gegenwart einer Verbindung X-Hal, in welcher X und Hal jeweils die oben genannten Bedeutungen haben.

Da sowohl X als auch Hal für Halogen stehen, handelt es sich um eine Interhalogenverbindung. X und Hal müssen nicht notwendigerweise für das gleiche Halogen stehen. Beispielsweise kann X für Iod oder Brom stehen und Hal für Chlor, Brom oder Iod. Vorzugsweise handelt es sich bei der Verbindung X-Hal aber um ein elementares Halogen, insbesondere F₂, Cl₂, Br₂ oder I₂. Besonders bevorzugt sind I₂ oder Br₂, ganz besonders bevorzugt ist I₂.

Vorzugsweise wird die Verbindung X-Hal in dem erfindungsgemäßen Verfahren in einer Gesamtmenge von 0,5 bis 10,0 Äquivalenten, vorzugsweise von 0,8 bis 5 Äquivalenten, weiter bevorzugt von 1 bis 2,5 Äquivalenten und besonders bevorzugt von 1,0 bis 2,0 Äquivalenten, bezogen auf die Verbindung Q-H, eingesetzt.

Die erfindungsgemäße Umsetzung der Verbindungen Q-H zu Verbindungen der Formel (IIIa) beziehungsweise (IIIb) und weiter zu Verbindungen der Formel (II) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrilidon (NEP), N-Butyl-2-pyrrilidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid.Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.

Besonders bevorzugte Lösungsmittel sind THF und N,N-Dimethylformamid (DMF), ganz besonders bevorzugt ist THF.

Das Lösungsmittel kann ferner entgast (sauerstoff-frei) sein.

Vorzugsweise wird für beide Verfahrensschritte a) und b) das gleiche Lösungsmittel verwendet. Alternative erfindungsgemäße Ausgestaltungen, in denen für die Verfahrensschritte a) und b) unterschiedliche Lösungsmittel verwendet werden, sind allerdings ebenfalls möglich, wobei die Lösungsmittel dann ebenfalls vorzugsweise aus den vorstehend genannten Lösungsmitteln ausgewählt sind und die jeweiligen als bevorzugt, besonders bevorzugt und ganz besonders bevorzugt angegebenen Lösungsmittel auf den jeweiligen Verfahrensschritt a) oder b) zu beziehen sind.

Die Umsetzung in Verfahrensschritt a) wird im Allgemeinen bei einer Temperatur zwischen 0°C und 80°C und zunehmend bevorzugt zwischen 10°C und 70°C, zwischen 15°C und 60°C, zwischen 20°C und 50°C, zwischen 20°C und 40°C und ganz besonders bevorzugt zwischen 20°C und 35°C, beispielsweise bei Raumtemperatur bzw. 25°C, durchgeführt.

Die Umsetzung in Verfahrensschritt b) wird im Allgemeinen bei einer Temperatur zwischen 0°C und 40°C und zunehmend bevorzugt zwischen 0°C und 35°C, zwischen 0°C und 30°C und ganz besonders bevorzugt zwischen 0°C und 25°C, beispielsweise bei Raumtemperatur bzw. 25°C, durchgeführt. Besonders vorteilhaft ist es, wenn Umsetzungen mit elementarem Brom (X und Hal stehen jeweils für Brom) bei 0°C erfolgen und Umsetzungen mit elementarem Iod (X und Hal stehen jeweils für Iod) bei Raumtemperatur bzw. 25°C erfolgen.

Die Reaktion wird üblicherweise bei Normaldruck durchgeführt, kann aber auch bei erhöhtem bzw. vermindertem Druck durchgeführt werden.

Die Isolierung der gewünschten Verbindungen der Formel (II) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

Zwei Beispiele von besonders bevorzugten Ausführungsformen des erfindungsgemäßen Verfahrens können anhand der folgenden Schemata (IIa) und (IIb) erläutert werden:

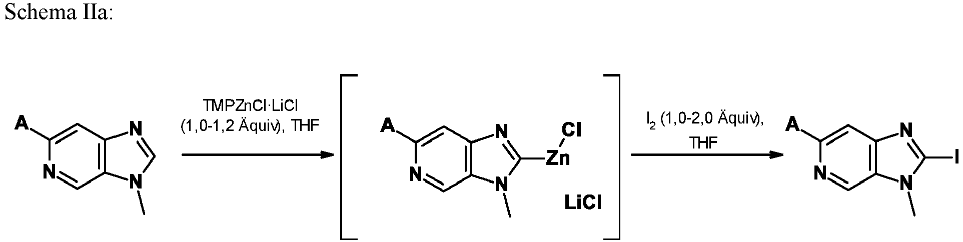

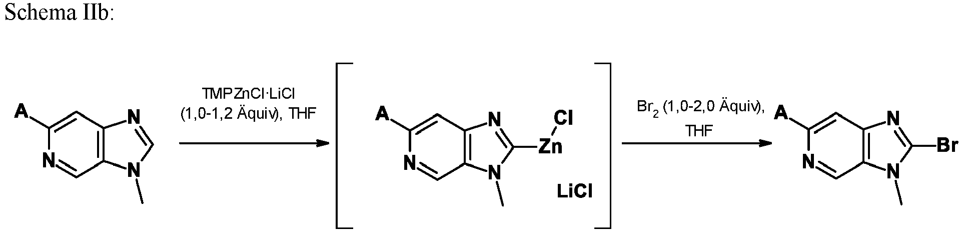

Schema IIa und Schema IIb unterscheiden sich lediglich dadurch, dass die Umsetzung in Verfahrensschritt b) mit elementarem Iod (IIa) beziehungsweise mit elementarem Brom (IIb) erfolgt. In beiden Schemata hat A jeweils die vorstehend angegebenen Bedeutungen. Die in Klammern angegebene Verbindung stellt die entsprechende Zwischenstufe der Formel IIIa dar, welche weiter zum Produkt, einer Verbindung der Formel (II), umgesetzt wird. Beide Umsetzungen finden in THF als Lösungsmittel statt. "Äquiv" bezeichnet die eingesetzte Menge an Äquivalenten TMPZnCl·LiCl bzw. Verbindung X-Hal, hier also elementarem Iod beziehungsweise elementarem Brom.

Weiterhin hierin beschrieben sind Verbindungen der Struktur Q-H, die ausgewählt sind aus den folgenden Verbindungen:

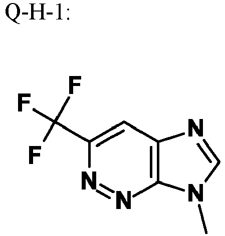

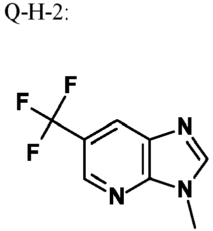

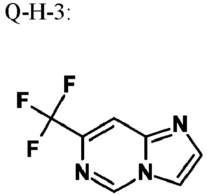

Weiterhin hierin beschrieben sind Verbindungen der Formel (IIIa), die ausgewählt sind aus den folgenden Verbindungen:

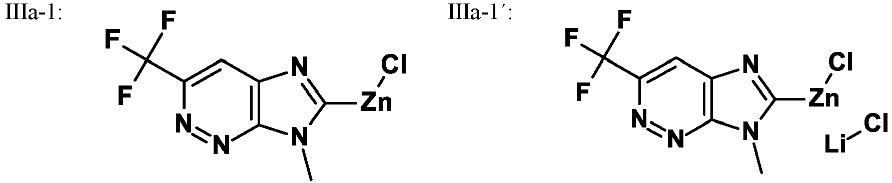

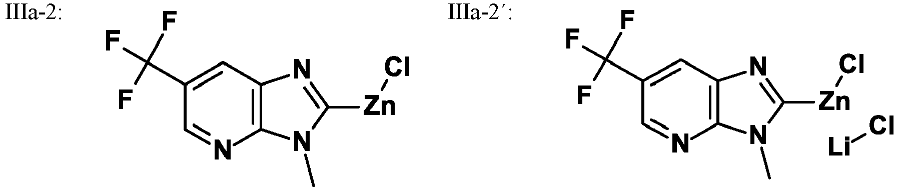

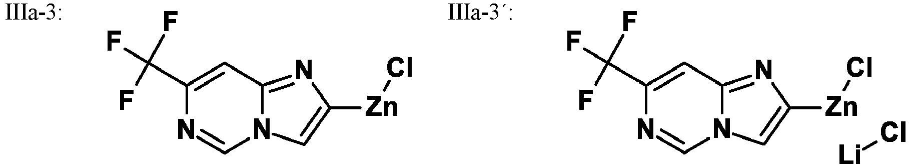

Ein weiterer Gegenstand der vorliegenden Erfindung sind Verbindungen der Formel (II)

Q-Hal (II),

in welcher (Ausgestaltung Q-Hal-1-1)
- Q: für ein Strukturelement aus der Reihe Q2, Q3, Q10, Q12 oder Q14 steht,
- R⁷: für (C₁-C₄)Alkyl oder (C₁-C₄)Alkoxy-(C₁-C₄)alkyl steht,
- A: für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht, und
- Hal: für Iod oder Brom steht.

### (Ausgestaltung Q-Hal-4-1)

- Q: steht ganz besonders bevorzugt für das Strukturelement Q2, Q3, Q12 oder Q14,
- R⁷: steht ganz besonders bevorzugt für Methyl, Ethyl, n-Propyl oder iso-Propyl, insbesondere Methyl,
- A: steht ganz besonders bevorzugt für Trifluormethyl, und
- Hal: steht ganz besonders bevorzugt für Iod oder Brom.

Die vorstehend aufgeführten Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

Beispiele für derartige ganz besonders bevorzugte Verbindungen sind:

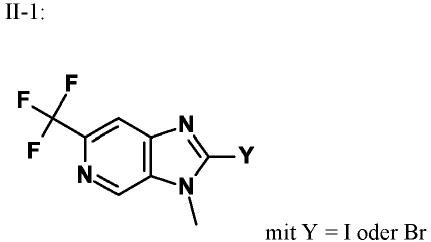

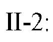

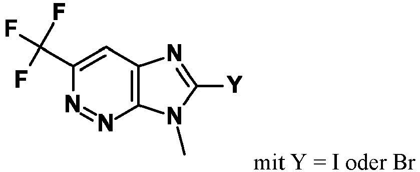

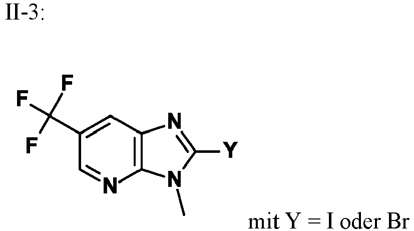

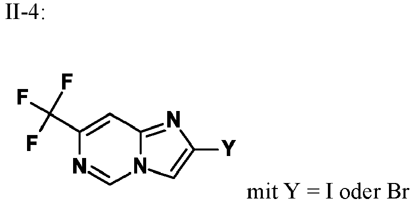

Die vorliegende Erfindung wird anhand der nachfolgenden Beispiele näher erläutert, wobei die Beispiele nicht in einer die Erfindung einschränkenden Weise zu interpretieren sind.

### Beispiel 1

### Synthese von 3-Methyl-6-(trifluortnethyl)-3H-imidazo[4,5-c]pyridin (Edukt)

N3-Methyl-6-(trifluormethyl)pyridin-3,4-diamin (500 mg, 2,6 mmol), gelöst in Ameisensäure (4 ml, 106 mmol), wurde mit Mikrowellen für 1 Stunde bei 150°C erhitzt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 3-Methyl-6-(trifluormethyl)-3H-imidazo[4,5-c]pyridin (480 mg, 91%) als weißen Feststoff. HPLC-MS: logP = 1,09; Masse (m/z+1): 202,0; 1HNMR (D6-DMSO): δ 9,14 (s, 1H), 8,61 (s, 1H), 8,19 (s, 1H), 4,02 (s, 3H).

### Beispiel 2

### Synthese von 2-Iod-3-methyl-6-(trifluormethyl)-3H-imidazo[4,5-c]pyridin:

3-Methyl-6-(trifluormethyl)-3H-imidazo[4,5-c]pyridin (201 mg, 1,0 mmol), gelöst in THF (1 ml), wurde mit TMPZnCl·LiCl (1,31 M in THF, 0,82 ml, 1,1 mmol) bei 25°C unter Argon versetzt; diese Reaktionslösung wurde 10 min gerührt. Anschließend wurde Iod (508 mg, 2,0 mmol in 2 ml THF) bei 25°C zugegeben und die Lösung während 20 min weitergerührt. Nach üblicher Aufarbeitung durch Zugabe von gesättigter Ammoniumchlorid und Natriumthiosulfat-Lösungen wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 2-Iod-3-methyl-6-(trifluormethyl)-3H-imidazo[4,5-c]pyridin (301 mg, 93%) als gelben Feststoff. HPLC-MS: logP = 1,77; Masse (m/z+1): 327,9; 1HNMR (D6-DMSO): δ 9,13 (s, 1H), 8,12 (s, 1H), 3,94 (s, 3H).

### Beispiel 3

### Synthese von 2-Brom-3-methyl-6-(trifluormethyl)-3H-imidazo[4.5-c]pyridin:

3-Methyl-6-(trifluormethyl)-3H-imidazo[4,5-c]pyridin (201 mg, 1,0 mmol), gelöst in THF (0,8 ml), wurde mit TMPZnCl·LiCl (1,35 M in THF, 0,82 ml, 1,1 mmol) bei 25°C unter Argon versetzt; diese Reaktionslösung wurde 10 min gerührt. Anschließend wurde Brom (224 mg, 1,4 mmol) bei 0°C zugegeben und die Lösung während 20 min weitergerührt. Nach üblicher Aufarbeitung durch Zugabe von gesättigter Ammoniumchlorid und Natriumthiosulfat-Lösungen wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 2-Brom-3-methyl-6-(trifluormethyl)-3H-imidazo[4,5-c]pyridin (269 mg, 96%) als gelben Feststoff. HPLC-MS: logP = 1,71; Masse (m/z+1): 281,0; 1HNMR (D6-DMSO): δ 9,15 (s, 1H), 8,17 (s, 1H), 3,96 (s, 3H).

### Beispiel 4

### Synthese von 7-Methyl-3-(trifluormethyl)-7H-imidazo[4.5-c]pyridazin (Edukt)

N3-Methyl-6-(trifluormethyl)pyridazin-3,4-diamin (1,0 g, 5,2 mmol), gelöst in Ameisensäure (5 ml, 132 mmol), wurde mit Mikrowellen für 1 Stunde bei 150°C erhitzt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 7-Methyl-3-(trifluormethyl)-7H-imidazo[4,5-c]pyridazin (758 mg, 73%) als weißen Feststoff. HPLC-MS: logP = 0,91; Masse (m/z+1): 203,1; 1HNMR (D6-DMSO): δ 8,92 (s, 1H), 8,62 (s, 1H), 4,08 (s, 3H).

### Beispiel 5

### Synthese von 6-Iod-7-methyl-3-(trifluormethyl)-7H-imidazo[4.5-c]pyridazin:

7-Methyl-3-(trifluormethyl)-7H-imidazo[4,5-c]pyridazin (203 mg, 1,0 mmol), gelöst in THF (0,8 ml), wurde mit TMPZnCl·LiCl (1,35 M in THF, 0,82 ml, 1,1 mmol) bei 25°C unter Argon versetzt; diese Reaktionslösung wurde 10 min gerührt. Anschließend wurde Iod (508 mg in 4 ml THF) bei 25°C zugegeben und die Lösung während 20 min weitergerührt. Nach üblicher Aufarbeitung durch Zugabe von gesättigter Ammoniumchlorid und Natriumthiosulfat -Lösungen wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 6-Iod-7-methyl-3-(trifluormethyl)-7H-imidazo[4,5-c]pyridazin (230 mg, 70%) als gelben Feststoff. HPLC-MS: logP = 1,66; Masse (m/z+1): 329,0; 1HNMR (D6-DMSO): δ 8,53 (s, 1H), 3,99 (s, 3H).

### Beispiel 6

### Synthese von 3-Methyl-6-(trifluormethyl)-3H-imidazo[4.5-b]pyridin (Edukt):

N2-Methyl-5-(trifluormethyl)pyridin-2,3-diamin (500 mg, 2,61 mmol), gelöst in Ameisensäure (4 ml, 106 mmol), wurde mit Mikrowellen für 1 Stunde bei 150°C erhitzt. Nach üblicher Aufarbeitung durch Zugabe von gesättigter Ammoniumchlorid-Lösung wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 3-Methyl-6-(trifluormethyl)-3H-imidazo[4,5-b]pyridin (385 mg, 74%) als weißen Feststoff. HPLC-MS: logP = 1,44; Masse (m/z+1): 202,1; 1HNMR (D6-DMSO): δ 8,77 (s, 1H), 8,67 (s, 1H), 8,52 (s, 1H), 3,90 (s, 3H).

### Beispiel 7

### Synthese von 2-Iod-3-methyl-6-(trifluormethyl)-3H-imidazo[4.5-b]pyridin:

3-Methyl-6-(trifluormethyl)-3H-imidazo[4,5-b]pyridin (100 mg, 0,49 mmol), gelöst in THF (0,4 ml), wurde mit TMPZnCl·LiCl (1,35 M in THF, 0,41 ml, 0,54 mmol) bei 25°C unter Argon versetzt; diese Reaktionslösung wurde 10 min gerührt. Anschließend wurde Iod (252 mg in 1 ml THF) bei 25°C zugegeben und die Lösung während 20 min weitergerührt. Nach üblicher Aufarbeitung durch Zugabe von gesättigter Ammoniumchlorid und Natriumthiosulfat-Lösungen wurde das Reaktionsgemisch mit Ethylacetat extrahiert, die vereinigten organischen Phasen über Na₂SO₄ getrocknet und im Membranpumpenvakuum eingeengt. Nach säulenchromatographischer Reinigung (Ethylacetat/Cyclohexan) erhielt man 2-Iod-3-methyl-6-(trifluormethyl)-3H-imidazo[4,5-b]pyridin (111 mg, 69%) als gelben Feststoff. HPLC-MS: logP = 2,29; Masse (m/z+1): 328,0; 1HNMR (D6-DMSO): δ 8,71 (s, 1H), 8,47 (s, 1H), 3,82 (s, 3H).

## Patentansprüche

1. Verfahren zur Herstellung von Verbindungen der Formel (II)
Q-Hal (II),
in welcher
Q für ein Strukturelement 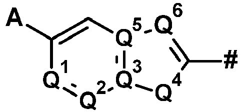
steht, wobei das Zeichen # die Bindung zu Hal anzeigt und
Q¹ für N oder CR⁶ steht,
Q² für N oder CR⁶ steht,
Q³ für N oder C steht,
Q⁴ für O, S, N oder NR⁷ steht,
Q⁵ für N oder C steht,
Q⁶ für N oder CH steht,
und wobei Q für ein Strukturelement aus der Reihe Q2, Q3, Q10, Q12 oder Q14 steht, 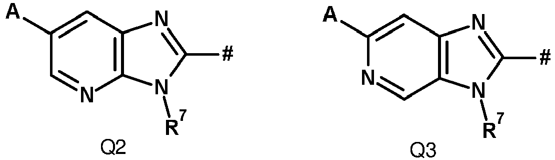 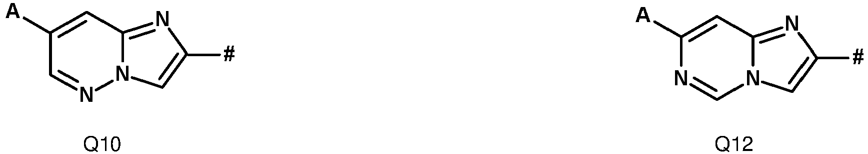 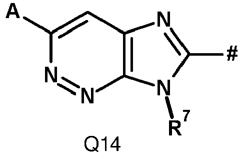
R⁶ für Wasserstoff, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht, und
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkylaminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl steht,
Hal für Halogen steht,
**dadurch gekennzeichnet, dass** in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 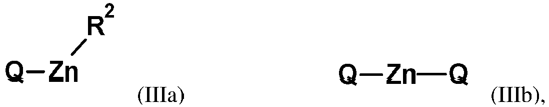
in welcher Q und R² jeweils die oben genannten Bedeutungen haben,
und diese Verbindung der Formel (IIIa) oder (IIIb) in einem zweiten Verfahrensschritt b) mit einer Verbindung der Struktur X-Hal, in welcher X für Halogen steht und Hal die oben genannte Bedeutung hat, zur Verbindung der Formel (II) umgesetzt wird.

2. Verfahren gemäß Anspruch 1, **dadurch gekennzeichnet, dass**
Q für ein Strukturelement aus der Reihe Q2, Q3, Q10, Q12 oder Q14 steht,
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl,
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
Hal und X dieselbe Bedeutung haben und für Fluor, Chlor, Iod oder Brom stehen, und
R² für Halogen, insbesondere Chlor, Brom oder Iod, steht.

3. Verfahren gemäß einem der Ansprüche 1 oder 2, **dadurch gekennzeichnet, dass**
Q für ein Strukturelement aus der Reihe Q2, Q3, Q10, Q12, oder Q14 steht,
R⁷ für (C₁-C₄)Alkyl oder (C₁-C₄)Alkoxy-(C₁-C₄)alkyl steht,
A für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
Hal und X dieselbe Bedeutung haben und für Iod oder Brom stehen, und
R² für Chlor steht.

4. Verfahren gemäß einem der Ansprüche 1 bis 3, **dadurch gekennzeichnet, dass**
Q für das Strukturelement Q2, Q3, Q12 oder Q14 steht,
R⁷ für Methyl, Ethyl, n-Propyl oder iso-Propyl, insbesondere Methyl, steht,
A für Trifluormethyl steht,
Hal und X dieselbe Bedeutung haben und für Iod oder Brom stehen, und
R² für Chlor steht.

5. Verfahren gemäß einem der Ansprüche 1 bis 4, **dadurch gekennzeichnet, dass** R³ und R⁴ gemeinsam eine -(CH₂)₅-Gruppe bilden, die durch 4 Methylgruppen substituiert ist.

6. Verfahren gemäß einem der Ansprüche 1 bis 5, **dadurch gekennzeichnet, dass** es sich bei der Zink-metallorganischen Base um eine Verbindung der Formel (V) handelt
(V) (TMP)ₓ ZnCl₂₋ₓ ,
worin x für die Zahl 1 oder 2 steht.

7. Verfahren gemäß einem der Ansprüche 1 bis 6, **dadurch gekennzeichnet, dass** die Zink-metallorganische Base in Verbindung mit einem Alkali- oder Erdalkalihalogenid, vorzugsweise Lithiumchlorid oder Magnesiumchlorid, vorliegt.

8. Verfahren gemäß einem der Ansprüche 1 bis 7, **dadurch gekennzeichnet, dass** die Zink-metallorganische Base in einer Gesamtmenge von 0,5 bis 5 Äquivalenten, bezogen auf die Verbindung Q-H, eingesetzt wird.

9. Verfahren gemäß einem der Ansprüche 1 bis 8, **dadurch gekennzeichnet, dass** es sich bei der Verbindung X-Hal um ein elementares Halogen, insbesondere um F₂, Cl₂, Br₂ oder I₂, handelt.

10. Verfahren gemäß einem der Ansprüche 1 bis 9, **dadurch gekennzeichnet, dass** die Verbindung X-Hal in einer Gesamtmenge von 0,5 bis 10,0 Äquivalenten, bezogen auf die Verbindung Q-H, eingesetzt wird.

11. Verfahren gemäß einem der Ansprüche 1 bis 10, **dadurch gekennzeichnet, dass** es in Gegenwart eines Lösungsmittels durchgeführt wird, welches ausgewählt ist aus der Gruppe bestehend aus Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrilidon (NEP), N-Butyl-2-pyrrilidon (NBP); N,N'-Dimethylpropylenharnstoff (DMPU), Halogenkohlenwasserstoff, aromatischer Kohlenwasserstoff, Chlorkohlenwasserstoff, Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, 1,2-Dichlorbenzol, Chlortoluol, Trichlorbenzol; 4-Methoxybenzol, fluoriertes Aliphat, fluorierter Aromat, Trichlortrifluorethan, Benzotrifluorid und 4-Chlorbenzotrifluorid, oder einer Mischung aus mindestens zwei dieser Lösungsmittel untereinander.

12. Verfahren gemäß Anspruch 11, **dadurch gekennzeichnet, dass** das Lösungsmittel THF oder N,N-Dimethylformamid (DMF) ist.

13. Verfahren gemäß einem der Ansprüche 1 bis 12, **dadurch gekennzeichnet, dass** Verfahrensschritt a) bei einer Temperatur zwischen 0°C und 80°C durchgeführt wird.

14. Verfahren gemäß einem der Ansprüche 1 bis 13, **dadurch gekennzeichnet, dass** Verfahrensschritt b) bei einer Temperatur zwischen 0°C und 40°C durchgeführt wird.

15. Verbindung der Formel (II)
Q-Hal (II),
in welcher
Q für ein Strukturelement aus der Reihe Q2, Q3, Q10, Q12 oder Q14 gemäß Anspruch 1 steht,
R⁷ für (C₁-C₄)Alkyl oder (C₁-C₄)Alkoxy-(C₁-C₄)alkyl steht,
A für Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht, und
Hal für Iod oder Brom steht.

## Claims

1. Process for preparing compounds of the formula (II)
Q-Hal (II)
in which
Q is a structural element 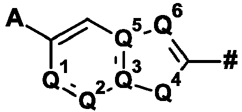
where the symbol # indicates the bond to Hal and
Q¹ is N or CR⁶,
Q² is N or CR⁶,
Q³ is N or C,
Q⁴ is O, S, N or NR⁷,
Q⁵ is N or C,
Q⁶ is N or CH,
Q is a structural element from the group of Q2, Q3, Q10, Q12 or Q14 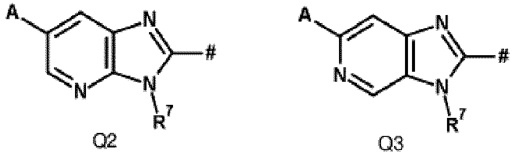 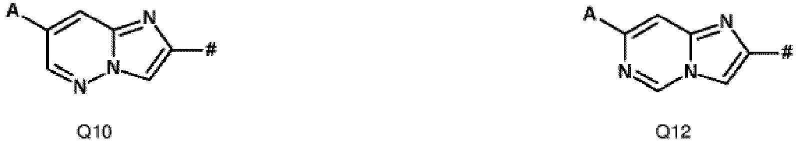 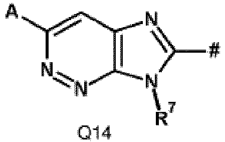
R⁶ is hydrogen, (C₁-C₄) alkyl, (C₁-C₄) haloalkyl, (C₁-C₄) cyanoalkyl, (C₁-C₄) hydroxyalkyl, (C₁-C₄)-alkoxy- (C₁-C₄) alkyl, (C₁-C₄) haloalkoxy- (C₁-C₄)-alkyl, (C₂-C₄) alkenyl, (C₂-C₄) alkenyloxy- (C₁-C₄)-alkyl, (C₂-C₄) haloalkenyloxy- (C₁-C₄) alkyl, (C₂-C₄) haloalkenyl, (C₂-C₄) cyanoalkenyl, (C₂-C₄)-alkynyl, (C₂-C₄) alkynyloxy- (C₁-C₄) alkyl, (C₂-C₄)-haloalkynyl, (C₃-C₆) cycloalkyl, (C₃-C₆)-cycloalkyl- (C₃-C₆) cycloalkyl, (C₁-C₄)-alkyl- (C₃-C₆) cycloalkyl, halo (C₃-C₆) cycloalkyl, (C₁-C₄) alkylthio- (C₁-C₄) alkyl, (C₁-C₄) alkylsulfinyl- (C₁-C₄) alkyl, (C₁-C₄)alkylsulfonyl- (C₁-C₄) alkyl or (C₁-C₄)-alkylcarbonyl- (C₁-C₄) alkyl,
R⁷ is (C₁-C₄) alkyl, (C₁-C₄) haloalkyl, (C₁-C₄)-cyanoalkyl, (C₁-C₄) hydroxyalkyl, (C₁-C₄)-alkoxy- (C₁-C₄) alkyl, (C₁-C₄) haloalkoxy- (C₁-C₄)-alkyl, (C₂-C₄) alkenyl, (C₂-C₄) alkenyloxy- (C₁-C₄)-alkyl, (C₂-C₄) haloalkenyloxy- (C₁-C₄) alkyl, (C₂-C₄) haloalkenyl, (C₂-C₄) cyanoalkenyl, (C₂-C₄)-alkynyl, (C₂-C₄) alkynyloxy- (C₁-C₄) alkyl, (C₂-C₄)-haloalkynyl, (C₃-C₆) cycloalkyl, (C₃-C₆)-cycloalkyl- (C₃-C₆) cycloalkyl, (C₁-C₄)-alkyl- (C₃-C₆) cycloalkyl, halo (C₃-C₆) cycloalkyl, (C₁-C₄) alkylthio- (C₁-C₄) alkyl, (C₁-C₄)-alkylsulfinyl- (C₁-C₄) alkyl, (C₁-C₄)alkylsulfonyl- (C₁-C₄) alkyl or (C₁-C₄)-alkylcarbonyl-(C₁-C₄)alkyl, and
A is hydrogen, cyano, halogen, (C₁-C₄) alkyl, (C₁-C₄) haloalkyl, (C₂-C₄) alkenyl, (C₂-C₄)-haloalkenyl, (C₂-C₄) alkynyl, (C₂-C₄) haloalkynyl, (C₃-C₆) cycloalkyl, (C₃-C₆) cycloalkyl- (C₃-C₆)-cycloalkyl, (C₁-C₄) alkyl- (C₃-C₆) cycloalkyl, (C₁-C₄) alkoxy, (C₁-C₄) haloalkoxy, (C₁-C₄)-alkoxyimino, (C₁-C₄) alkylthio, (C₁-C₄)-haloalkylthio, (C₁-C₄) alkylsulfinyl, (C₁-C₄)-haloalkylsulfinyl, (C₁-C₄) alkylsulfonyl, (C₁-C₄)-haloalkylsulfonyl, (C₁-C₄)alkylsulfonyloxy, (C₁-C₄) alkylcarbonyl, (C₁-C₄) haloalkylcarbonyl, aminocarbonyl, (C₁-C₄) alkylaminocarbonyl, di- (C₁-C₄) alkylaminocarbonyl, (C₁-C₄)alkylsulfonylamino, (C₁-C₄) alkylamino, di- (C₁-C₄)-alkylamino, aminosulfonyl, (C₁-C₄)-alkylaminosulfonyl or di-(C₁-C₄)-alkylaminosulfonyl,
Hal is halogen,
**characterized in that**, in a first process step a), a compound Q-H in which Q is as defined above
is reacted with an organozinc base of the structure (NR³R⁴)-Zn-R² or (NR³R⁴)₂-Zn in which
R² is halogen or -O-pivaloyl and
R³ and R⁴ together form a - (CH₂) ₄-, - (CH₂)₅- or - (CH₂) ₂O (CH₂) ₂- group, where each of these groups may optionally be substituted by 1, 2, 3 or 4 R⁵ radicals and R⁵ is selected from the group consisting of methyl, ethyl, n-propyl and i-propyl,
to give a compound of the formula (IIIa) or the formula (IIIb) 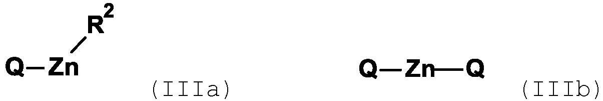 in which Q and R² each have the definitions given above,
and this compound of the formula (IIIa) or (IIIb) is reacted in a second process step b) with a compound of the structure X-Hal in which X is halogen and Hal has the abovementioned definition to give the compound of the formula (II).

2. Process according to Claim 1, **characterized in that**
Q is a structural element from the group of Q2, Q3, Q10, Q12 or Q14,
R⁷ is (C₁-C₄) alkyl, (C₁-C₄) haloalkyl, (C₁-C₄)-alkoxy- (C₁-C₄) alkyl, (C₁-C₄) haloalkoxy- (C₁-C₄)-alkyl, (C₁-C₄) alkylthio- (C₁-C₄) alkyl, (C₁-C₄)-alkylsulfinyl- (C₁-C₄) alkyl, (C₁-C₄)alkylsulfonyl- (C₁-C₄) alkyl or (C₁-C₄)-alkylcarbonyl- (C₁-C₄) alkyl,
A is fluorine, chlorine, bromine, fluoromethyl, difluoromethyl, trifluoromethyl, fluoroethyl (CH₂CFH₂, CHFCH₃) , difluoroethyl (CF₂CH₃, CH₂CHF₂, CHFCFH₂) , trifluoroethyl, (CH₂CF₃, CHFCHF₂, CF₂CFH₂) , tetrafluoroethyl (CHFCF₃, CF₂CHF₂) , pentafluoroethyl, trifluoromethoxy, difluorochloromethoxy, dichlorofluoromethoxy, trifluoromethylthio, trifluoromethylsulfinyl or trifluoromethylsulfonyl,
Hal and X have the same definition and are fluorine, chlorine, iodine or bromine, and
R² is halogen, especially chlorine, bromine or iodine.

3. Process according to either of Claims 1 and 2, **characterized in that**
Q is a structural element from the group of Q2, Q3, Q10, Q12, or Q14,
R⁷ is (C₁-C₄) alkyl or (C₁-C₄) alkoxy- (C₁-C₄) alkyl,
A is trifluoromethyl, fluoroethyl (CH₂CFH₂, CHFCH₃) , difluoroethyl (CF₂CH₃, CH₂CHF₂, CHFCFH₂) , trifluoroethyl, (CH₂CF₃, CHFCHF₂, CF₂CFH₂) , tetrafluoroethyl (CHFCF₃, CF₂CHF₂) , pentafluoroethyl, trifluoromethylthio, trifluoromethylsulfinyl or trifluoromethylsulfonyl,
Hal and X have the same definition and are iodine or bromine, and
R² is chlorine.

4. Process according to any of Claims 1 to 3, **characterized in that**
Q is the structural element Q2, Q3, Q12 or Q14,
R⁷ is methyl, ethyl, n-propyl or isopropyl, especially methyl,
A is trifluoromethyl,
Hal and X have the same definition and are iodine or bromine, and
R² is chlorine.

5. Process according to any of Claims 1 to 4, **characterized in that** R³ and R⁴ together form a -(CH₂)₅- group substituted by 4 methyl groups.

6. Process according to any of Claims 1 to 5, **characterized in that** the organozinc base is a compound of the formula (V)
(V) (TMP) ₓ ZnCl₂₋ₓ
in which x is the number 1 or 2.

7. Process according to any of Claims 1 to 6, **characterized in that** the organozinc base is present in conjunction with an alkali metal halide or alkaline earth metal halide, preferably lithium chloride or magnesium chloride.

8. Process according to any of Claims 1 to 7, **characterized in that** the organozinc base is used in a total amount of 0.5 to 5 equivalents, based on the compound Q-H.

9. Process according to any of Claims 1 to 8, **characterized in that** the compound X-Hal is an elemental halogen, especially F₂, Cl₂, Br₂ or I₂.

10. Process according to any of Claims 1 to 9, **characterized in that** the compound X-Hal is used in a total amount of 0.5 to 10.0 equivalents, based on the compound Q-H.

11. Process according to any of Claims 1 to 10, **characterized in that** it is conducted in the presence of a solvent selected from the group consisting of tetrahydrofuran (THF), 1,4-dioxane, diethyl ether, diglyme, methyl tert-butyl ether (MTBE), tert-amyl methyl ether (TAME), 2-methyl-THF, toluene, xylenes, mesitylene, ethylene carbonate, propylene carbonate, N,N-dimethylacetamide, N,N-dimethylformamide (DMF), N-methylpyrrolidone (NMP), N-ethyl-2-pyrrolidone (NEP), N-butyl-2-pyrrolidone (NBP); N,N'-dimethylpropyleneurea (DMPU), halohydrocarbon, aromatic hydrocarbon, chlorohydrocarbon, tetrachloroethylene, tetrachloroethane, dichloropropane, methylene chloride, dichlorobutane, chloroform, carbon tetrachloride, trichloroethane, trichloroethylene, pentachloroethane, difluorobenzene, 1,2-dichloroethane, chlorobenzene, bromobenzene, dichlorobenzene, 1,2-dichlorobenzene, chlorotoluene, trichlorobenzene; 4-methoxybenzene, fluorinated aliphatic, fluorinated aromatic, trichlorotrifluoroethane, benzotrifluoride and 4-chlorobenzotrifluoride, or a mixture of at least two of these solvents with one another.

12. Process according to Claim 11, **characterized in that** the solvent is THF or N,N-dimethylformamide (DMF).

13. Process according to any of Claims 1 to 12, **characterized in that** process step a) is conducted at a temperature between 0°C and 80°C.

14. Process according to any of Claims 1 to 13, **characterized in that** process step b) is conducted at a temperature between 0°C and 40°C.

15. Compound of the formula (II)
Q-Hal (II)
in which
Q is a structural element Q2, Q3, Q10, Q12 or Q14 according to Claim 1,
R⁷ is (C₁-C₄) alkyl or (C₁-C₄) alkoxy- (C₁-C₄) alkyl,
A is trifluoromethyl, fluoroethyl (CH₂CFH₂, CHFCH₃) , difluoroethyl (CF₂CH₃, CH₂CHF₂, CHFCFH₂) , trifluoroethyl, (CH₂CF₃, CHFCHF₂, CF₂CFH₂) , tetrafluoroethyl (CHFCF₃, CF₂CHF₂) , pentafluoroethyl, trifluoromethylthio, trifluoromethylsulfinyl or trifluoromethylsulfonyl, and
Hal is iodine or bromine.

## Revendications

1. Procédé pour la préparation de composés de formule (II)
Q-Hal (II),
dans laquelle
Q représente un élément structural 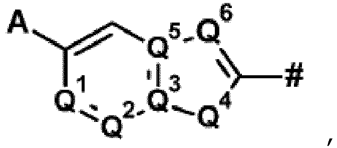
le symbole # indiquant la liaison à Hal et
Q¹ représentant N ou CR⁶,
Q² représentant N ou CR⁶,
Q3 représentant N ou C,
Q⁴ représentant O, S, N ou NR⁷,
Q⁵ représentant N ou C,
Q⁶ représentant N ou CH,
et Q représentant un élément structural de la série Q2, Q3, Q10, Q12 ou Q14, 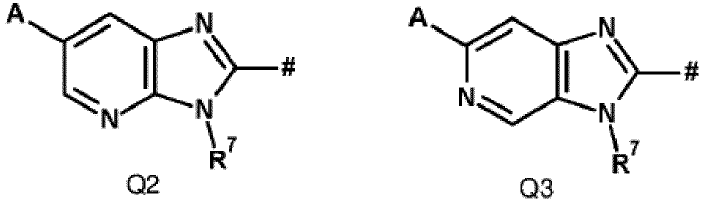 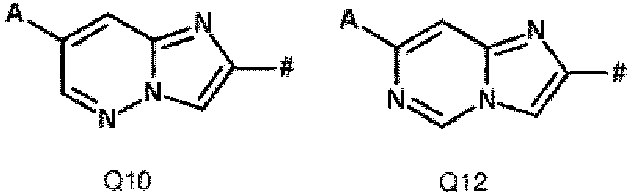 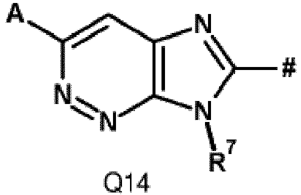
R⁶ représentant hydrogène, (C₁-C₄) alkyle, (C₁-C₄) halogénoalkyle, (C₁-C₄) cyanoalkyle, (C₁-C₄) hydroxyalkyle, (C₁-C₄) alcoxy- (C₁-C₄) alkyle, (C₁-C₄) halogénoalcoxy- (C₁-C₄) alkyle, (C₂-C₄) alcényle, (C₂-C₄) alcényloxy- (C₁-C₄) alkyle, (C₂-C₄) halogénoalcényloxy- (C₁-C₄) alkyle, (C₂-C₄) halogénoalcényle, (C₂-C₄) cyanoalcényle, (C₂-C₄) alcynyle, (C₂-C₄) alcynyloxy- (C₁-C₄) alkyle, (C₂-C₄) halogénoalcynyle, (C₃-C₆) cycloalkyle, (C₃-C₆) cycloalkyl- (C₃-C₆) cycloalkyle, (C₁-C₄)alkyl-(C₃-C₆) cycloalkyle, halogéno (C₃-C₆) cycloalkyle, (C₁-C₄) alkylthio- (C₁-C₄) alkyle, (C₁-C₄) alkylsulfinyl- (C₁-C₄) alkyle, (C₁-C₄) alkylsulfonyl- (C₁-C₄) alkyle ou (C₁-C₄) alkylcarbonyl- (C₁-C₄) alkyle,
R⁷ représentant (C₁-C₄) alkyle, (C₁-C₄) halogénoalkyle, (C₁-C₄) cyanoalkyle, (C₁-C₄) hydroxyalkyle, (C₁-C₄) alcoxy- (C₁-C₄) alkyle, (C₁-C₄) halogénoalcoxy- (C₁-C₄) alkyle, (C₂-C₄) alcényle, (C₂-C₄) alcényloxy- (C₁-C₄) alkyle, (C₂-C₄) halogénoalcényloxy- (C₁-C₄) alkyle, (C₂-C₄) halogénoalcényle, (C₂-C₄) cyanoalcényle, (C₂-C₄) alcynyle, (C₂-C₄) alcynyloxy- (C₁-C₄) alkyle, (C₂-C₄) halogénoalcynyle, (C₃-C₆) cycloalkyle, (C₃-C₆) cycloalkyl- (C₃-C₆) cycloalkyle, (C₁-C₄)alkyl-(C₃-C₆) cycloalkyle, halogéno (C₃-C₆) cycloalkyle, (C₁-C₄) alkylthio- (C₁-C₄) alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle, et
A représentant hydrogène, cyano, halogène, (C₁-C₄)alkyle, (C₁-C₄)halogénoalkyle, (C₂-C₄)alcényle, (C₂-C₄)halogénoalcényle, (C₂-C₄)alcynyle, (C₂-C₄)halogénoalcynyle, (C₃-C₆)cycloalkyle, (C₃-C₆)cycloalkyl-(C₃-C₆)cycloalkyle, (C₁-C₄)alkyl- (C₃-C₆)cycloalkyle, (C₁-C₄)alcoxy, (C₁-C₄)halogénoalcoxy, (C₁-C₄)alcoxyimino, (C₁-C₄)alkylthio, (C₁-C₄)halogénoalkylthio, (C₁-C₄)alkylsulfinyle, (C₁-C₄)halogénoalkylsulfinyle, (C₁-C₄)alkylsulfonyle, (C₁-C₄)halogénoalkylsulfonyle, (C₁-C₄)alkylsulfonyloxy, (C₁-C₄)alkylcarbonyle, (C₁-C₄)halogénoalkylcarbonyle, aminocarbonyle, (C₁-C₄) alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle ou di-(C₁-C₄)alkylaminosulfonyle,
Hal représentant halogène,
**caractérisé en ce que** dans une première étape de procédé a), un composé Q-H, dans lequel Q possède la signification mentionnée ci-dessus,
est transformé avec une base organométallique au zinc de structure (NR³R⁴)-Zn-R² ou (NR³R⁴)₂-Zn, dans laquelle
R² représente halogène ou -O-pivaloyle et
R³ et R⁴ représentent ensemble un groupe -(CH₂)₄-, - (CH₂)₅- ou - (CH₂)₂O(CH₂)₂-, chacun de ces groupes pouvant éventuellement être substitué par 1, 2, 3 ou 4 radicaux R⁵ et R⁵ étant choisi dans le groupe constitué par méthyle, éthyle, n-propyle et i-propyle,
pour donner un composé de formule (IIIa) ou de formule (IIIb), 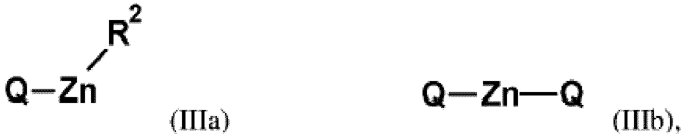 dans lesquelles Q et R² possèdent à chaque fois les significations mentionnées ci-dessus,
et ce composé de formule (IIIa) ou (IIIb) est transformé dans une deuxième étape de procédé b) avec un composé de structure X-Hal, dans laquelle X représente halogène et Hal possède la signification mentionnée ci-dessus, pour donner le composé de formule (II).

2. Procédé selon la revendication 1, **caractérisé en ce que** Q représente un élément structural de la série Q2, Q3, Q10, Q12 ou Q14,
R⁷ représente (C₁-C₄)alkyle, (C₁-C₄)halogénoalkyle, (C₁-C₄)alcoxy- (C₁-C₄)alkyle, (C₁-C₄)halogénoalcoxy-(C₁-C₄)alkyle, (C₁-C₄)alkylthio-(C₁-C₄)alkyle, (C₁-C₄)alkylsulfinyl-(C₁-C₄)alkyle, (C₁-C₄)alkylsulfonyl- (C₁-C₄)alkyle ou (C₁-C₄)alkylcarbonyl-(C₁-C₄)alkyle,
A représente fluor, chlore, brome, fluorométhyle, difluorométhyle, trifluorométhyle, fluoroéthyle (CH₂CFH₂, CHFCH₃), difluoroéthyle (CF₂CH₃, CH₂CHF₂, CHFCFH₂), trifluoroéthyle, (CH₂CF₃, CHFCHF₂, CF₂CFH₂), tétrafluoroéthyle (CHFCF₃, CF₂CHF₂), pentafluoroéthyle, trifluorométhoxy, difluorchlorméthoxy, dichlorfluorométhoxy, trifluorométhylthio, trifluorométhylsulfinyle ou trifluorométhylsulfonyle,
Hal et X possèdent la même signification et représentent fluor, chlore, iode ou brome, et
R² représente halogène, en particulier chlore, brome ou iode.

3. Procédé selon l'une quelconque des revendications 1 et 2, **caractérisé en ce que**
Q représente un élément structural de la série Q2, Q3, Q10, Q12 ou Q14,
R⁷ représente (C₁-C₄)alkyle ou (C₁-C₄)alcoxy- (C₁-C₄)alkyle,
A représente trifluorométhyle, fluoroéthyle (CH₂CFH₂, CHFCH₃), difluoroéthyle (CF₂CH₃, CH₂CHF₂, CHFCFH₂), trifluoroéthyle, (CH₂CF₃, CHFCHF₂, CF₂CFH₂), tétrafluoroéthyle (CHFCF₃, CF₂CHF₂), pentafluoroéthyle, trifluorométhylthio, trifluorométhylsulfinyle ou trifluorométhylsulfonyle,
Hal et X possèdent la même signification et représentent iode ou brome, et
R² représente chlore.

4. Procédé selon l'une quelconque des revendications 1 à 3, **caractérisé en ce que**
Q représente l'élément structural Q2, Q3, Q12 ou Q14,
R⁷ représente méthyle, éthyle, n-propyle ou iso-propyle, en particulier méthyle,
A représente trifluorométhyle,
Hal et X possèdent la même signification et représentent iode ou brome, et
R² représente chlore.

5. Procédé selon l'une quelconque des revendications 1 à 4, **caractérisé en ce que** R³ et R⁴ forment ensemble un groupe -(CH₂)₅- qui est substitué par 4 groupes méthyle.

6. Procédé selon l'une quelconque des revendications 1 à 5, **caractérisé en ce que** la base organométallique au zinc est un composé de formule (V)
**(V)** **(TMP)ₓZnCl₂₋ₓ** ,
dans laquelle x représente le nombre 1 ou 2.

7. Procédé selon l'une quelconque des revendications 1 à 6, **caractérisé en ce que** la base organométallique au zinc est présente en association avec un halogénure alcalin ou alcalino-terreux, de préférence le chlorure de lithium ou le chlorure de magnésium.

8. Procédé selon l'une quelconque des revendications 1 à 7, **caractérisé en ce que** la base organométallique au zinc est utilisée en une quantité totale de 0,5 à 5 équivalents, par rapport au composé Q-H.

9. Procédé selon l'une quelconque des revendications 1 à 8, **caractérisé en ce que** le composé X-Hal est un halogène élémentaire, en particulier F₂, Cl₂, Br₂ ou I₂.

10. Procédé selon l'une quelconque des revendications 1 à 9, **caractérisé en ce que** le composé X-Hal est utilisé en une quantité totale de 0,5 à 10,0 équivalents, par rapport au composé Q-H.

11. Procédé selon l'une quelconque des revendications 1 à 10, **caractérisé en ce qu'**il est mis en œuvre en présence d'un solvant qui est choisi dans le groupe constitué par le tétrahydrofuranne (THF), le 1,4-dioxanne, le diéthyléther, le diglyme, le méthyl-tert-butyléther (MTBE), le tert-amyl-méthyléther (TAME), le 2-méthyl-THF, le toluène, des xylènes, le mésitylène, le carbonate d'éthylène, le carbonate de propylène, le N,N-diméthylacétamide, le N,N-diméthylformamide (DMF), la N-méthylpyrrolidone (NMP), la N-éthyl-2-pyrrolidone (NEP), la N-butyl-2-pyrrolidone (NBP) ; la N,N'-diméthylpropylènurée (DMPU), un hydrocarbure halogéné, un hydrocarbure aromatique, un hydrocarbure chloré, le tétrachloroéthylène, le tétrachloroéthane, le dichloropropane, le chlorure de méthylène, le dichlorobutane, le chloroforme, le tétrachlorure de carbone, le trichloroéthane, le trichloréthylène, le pentachloroéthane, un difluorobenzène, le 1,2-dichloroéthane, le chlorobenzène, le bromobenzène, un dichlorobenzène, le 1,2-dichlorobenzène, un chlorotoluène, un trichlorobenzène ; le 4-méthoxybenzène, un composé aliphatique fluoré, un composé aromatique fluoré, un trichlorotrifluoroéthane, le trifluorométhylbenzène et le 4-chlorotrifluorométhylbenzène, ou un mélange d'au moins deux de ces solvants.

12. Procédé selon la revendication 11, **caractérisé en ce que** le solvant est le THF ou le N,N-diméthylformamide (DMF).

13. Procédé selon l'une quelconque des revendications 1 à 12, **caractérisé en ce que** l'étape de procédé a) est réalisée à une température comprise entre 0 °C et 80 °C.

14. Procédé selon l'une quelconque des revendications 1 à 13, **caractérisé en ce que** l'étape de procédé b) est réalisée à une température comprise entre 0 °C et 40 °C.

15. Composé de formule (II)
**Q-Hal** **(II),**
dans laquelle
Q représente un élément structural de la série Q2, Q3, Q10, Q12 ou Q14 selon la revendication 1,
R⁷ représente (C₁-C₄)alkyle ou (C₁-C₄)alcoxy-(C₁-C₄)alkyle,
A représente trifluorométhyle, fluoroéthyle (CH₂CFH₂, CHFCH₃), difluoroéthyle (CF₂CH₃, CH₂CHF₂, CHFCFH₂), trifluoroéthyle, (CH₂CF₃, CHFCHF₂, CF₂CFH₂), tétrafluoroéthyle (CHFCF₃, CF₂CHF₂), pentafluoroéthyle, trifluorométhylthio, trifluorométhylsulfinyle ou trifluorométhylsulfonyle, et
Hal représente iode ou brome.
